# EUROPÄISCHE PATENTSCHRIFT

(11) **EP 1 934 595 B1**
(45) Veröffentlichungstag und Bekanntmachung des Hinweises auf die Patenterteilung: **26.06.2013**
(21) Anmeldenummer: 06828482.7
(22) Anmeldetag: 28.09.2006
(51) Int. Cl.: G01N 33/10, G01N 11/14

(54) **Verfahren und Vorrichtung zum Schnelltest der Qualität von Getreiden, Schroten und Mehlen durch Messung der Glutenaggregation**
Method and device for rapid testing of the quality of cereals, grits and flours by measuring the aggregation of gluten
Procédé et appareil pour tester rapidement la qualité de céréales, de gruaux et de farines en mesurant l'agrégation du gluten

(30) Priorität: 07.10.2005 DE 102005048184
(43) Veröffentlichungstag der Anmeldung: 25.06.2008
(73) Patentinhaber: Brabender GmbH & Co. KG, 47055 Duisburg (DE)
(72) Erfinder: DREISÖRNER, Jens, 32312 Lübbecke (DE)
(74) Vertreter: Röther, Peter
(86) Internationale Anmeldenummer: PCT/DE2006/001718
(87) Internationale Veröffentlichungsnummer: WO 2007/041987

(56) Entgegenhaltungen:
- DD-A1- 233 423
- DE-A1- 19 828 667
- GB-A- 1 217 329
- DATABASE WPI Week 198844 Derwent Publications Ltd., London, GB; AN 1988-313671 XP002435733 -& SU 1 392 453 A (ODESS FOOD IND TECH) 30. April 1988 (1988-04-30)

## Beschreibung

Die Erfindung betrifft ein Verfahren zum Schnelltest der Qualität von Getreiden, Schroten und Mehlen durch Messung des Glutenaggregation.

In ihrem Artikel "Qualitätsbeschreibung von Mehlen für nicht hefegelockerte feine Backwaren" aus; Getreide, Mehl und Brot 52 (1998) 2" führen die Autoren Botterbrodt, Hanneforth, Lewandrowski und Brümmer aus, dass bei der Beschreibung der Mehlqualität die Fragen, welche Kenndaten die Qualität charakterisieren und welche Kenndaten für den Einsatzbereich Aussagekraft besitzen, im Vordergrund stehen. Die Kennzahlen der Mehlqualität, z.B. der Proteingehalt, der Feuchtklebergehalt, aber auch von Backversuchen wie z.B. des Rapid-Mix-Testes (RMT), sind dafür bekannte Größen.

In dem Artikel wird zum Schnelltesten von Mehlen der sogenannte Knock-out-Test angeführt, der bereits im Jahre 1997 in der Zeitschrift "Getreide, Mehl und Brot", Ausgabe 51 von den Autoren Hanneforth, Zwingelberg und Gebhard zum ersten Mal beschrieben wurde. Bei diesem auch als Glutenaggregationstest bekannten Test wird das Aggregationsverhalten einer Mehl-/Wassersuspension in einem Verhältnis von 1:1,2 bis 1:1,5 bei Verwendung von destilliertem Wasser beobachtet. Das zu untersuchende Getreide wird auf eine bestimmte Temperatur gebracht und in einem handelsüblichen Mixer in den Mixaufsatz eingewogen (beispielsweise 110 g). Das Wasser wird auf beispielsweise 23°C temperiert und ebenfalls in den Mixer eingewogen (beispielsweise 132 g).

Der Mixer wird eingeschaltet und auf einer mittleren Stufe(bei etwa 4500 U/min) etwa 20 Sekunden laufengelassen. Danach wird der Mixeraufsatz angekratzt und anschließend wird der Mixer nochmals 20 Sekunden auf der vorherigen Stufe betrieben. Sodann wird der Mixer kurz angehalten, und die eigentliche Messung beginnt, wobei der Mixer auf einer höheren Stufe läuft (beispielsweise bei 6250 U/min). Die Messung wird aufgezeichnet und der Zeitraum zwischen Messbeginn und Messende wird erfasst. Das Ende der Messung ist erreicht, wenn die Stromaufnahme von 2 Ampere nach dem Maximum erreicht ist. Ziel und Zweck dieses Testes ist es, angelieferte und entsprechend bezeichnete Weizenpartien (z.B. der E-und A-Weizengruppen), durch ein entsprechendes Aggregationsverhalten zu bestätigen.

Zur Bestimmung des Zeitpunktes der maximalen Klebervernetzung wird ein Amperemeter an das Mixgerät angeschlossen. Durch die Bildung des Klebernetzwerkes in der Masse erfährt der Mixer einen steigenden Widerstand, der zu erhöhter Stromaufnahme und somit zum Anstieg der angezeigten Amperezahl führt.

Nachteilig bei diesem Verfahren ist jedoch die Tatsache, dass in verschiedenen Geräten niemals identische Elektromotoren vorhanden sein können, so dass jedes Gerät unterschiedliche Stromaufnahmen verzeichnet, da unterschiedliche Wicklungen auch unterschiedliche thermische Randbedingungen mit sich bringen.

In der DE 198 28 667 A1 ist ein Verfahren beschrieben, bei dem ebenfalls die Stromaufnahme gemessen wird, allerdings zu Zwecken der Viskositätsmessung. Darüber hinaus ist bei diesem vorbekannten Verfahren eine relativ hohe Temperatur (>50°C) der Probe notwendig.

Aus der SU 1 392 453 A1 ist ein Rotationsviskosimeter mit einem zylindrischer Rührkörper (ohne Ausnehmungen) bekannt, das zur Bestimmung rheologischer Eigenschaften von Lebensmitteln verwendet wird. Die bei der Rotation des Rührkörpers in der Probe entstehenden Scherkräfte erzeugen ein auf den Stator des drehbar gelagerten Motors wirkendes Drehmoment, welches über einen Verbindungsarm auf einen Dehnungsmessstreifen übertragen wird, um so die Viskosität bestimmen zu können.

Der Erfindung liegt die Aufgabe zugrunde, einen Schnelltest der eingangs genannten Art so zu führen, dass unabhängig vom Gerät bei minimierter Probenmenge auch bei schwachen Mehlen exaktere Ergebnisse erreicht werden.

Die Erfindung löst diese Aufgabe mit den Merkmalen des Patentanspruchs 1. Weitere vorteilhafte Ausgestaltungen der Erfindung sind Gegenstand der Unteransprüche, wobei zur Durchführung des Verfahrens eine besonders ausgestaltete Vorrichtung vorgeschlagen wird.

Die entscheidende Neuerung gegenüber dem Stand der Technik ist beim erfindungsgemäßen Verfahren darin zu sehen, dass direkt die zur Verformung erforderliche auftretende Kraft gemessen wird, die bei der Aggregation langsam zunimmt und dann ein Maximum erreicht. Somit ist man unabhängig von thermischen Effekten, die die Messung verfälschen können. Die Torsionskräfte, die die Probe während der Messung z.B. auf den drehbar gelagerten Elektromotor für das Rührpaddel ausübt, wird über einen definiert langen Hebelarm auf die mit Dehnungsmessstreifen arbeitende Messvorrichtung übertragen. Die den Messvorgang begleitende Software rechnet aus der Kraft und dem bekannten Hebelarm das entsprechende Drehmoment aus.

Mit Hilfe der erfindungsgemäßen Methode ist es möglich, unterschiedliche Mehle anhand ihrer Maxima zu differenzieren.

Bei einem Versuch wurden drei verschiedene Mehrsorten verwendet, wobei ein so genanntes starkes Mehl, ein so genanntes schwaches Mehr und ein Mehl, das zwischen beiden liegt, getestet.

Dabei erreichte das starke Mehl bereits nach 480 Sekunden das Maximum, während das schwache Mehl erst nach etwas über 900 Sekunden das Aggregationsmaximum erreicht hatte.

Mit Hilfe der erfindungsgemäßen Methode kann mit Einsatz von einer Lösung aus Mineralsalzen und Säuren/Basen das Aggregationsverhalten auch bei einem schwachen Mehl verifiziert werden, wobei hier das Maximum bei einer Aggregationszeit von etwa 430 Sekunden erreicht wurde. Demgegenüber zeigte das gleiche schwache Mehl in destilliertem Wasser kein Aggregationsverhalten.

Zusammenfassend kann gesagt werden, dass mit Hilfe des erfindungsgemäßen Verfahren unterschiedlichste Mehle, Schrote, Getreide und auch Backmischungen auf ihr Aggregationsverhalten hin sicher getestet werden können.

Hierzu ist nur eine geringe Testmenge notwendig. Versuche haben gezeigt, dass bei einem Verhältnis von zu testendem Material und Probenlösung zwischen 1:1,2 und 1:1,25 Probenmengen von 10 bis 20 g und in etwa ebenso große Lösungsmengen ausreichend sind.

Gemäß Anspruch 5 sollte das Suspensionsmedium eine Leitfähigkeit von > 5µS und ein pH-Wert < 2 bis 13 aufweisen.

Gemäß Anspruch 3 ist vorgesehen, dass die Drehzahl des Messpaddels 6000 U/min nicht überschreiten soll. Das ist zum einen schonender für die Probe und zum anderen auch schonender für das Gerät.

Die Drehzahl, die auch wesentlich niedriger als 6000 U/min liegen kann (zwischen 1000 und 6000 U/min), wird durch die Software vorgegeben und durch ständigen Vergleich der Soll-und Istwerte automatisch korrigiert.

Bei der Auswertung wird auch die kontinuierlich aufgezeichnete Temperatur berücksichtigt.

Eine bevorzugte Vorrichtung zur Durchführung des oben genannten Verfahrens ist durch den Anspruch 6 gegeben. Es handelt sich um ein Gerät, bei dem der Antrieb und das Rührpaddel oberhalb des Probenbechers angeordnet ist, so dass das Rührpaddel von oben in den Probenbecher eingefahren werden kann. Der als Antrieb des Rührpaddels dienende Elektromotor ist drehbar gelagert und mittels eines Hebelarms mit der Kraftmesseinrichtung verbunden. Die Kraftmesseinrichtung arbeitet mit Dehnungsmessstreifen. Die von der Probe auf das Rührpaddel und von dort auf den Elektromotor ausgeübte Torsionskraft wird über den Hebelarm auf die Dehnungsmessstreifen übertragen. Von der Software wird dann durch Produktbildung aus Kraft und Hebelarm das entsprechende Drehmoment errechnet. Besonders vorteilhaft ist die neue Geometrie des Probenbechers und des Rührpaddels, die nämlich in ihrer Form optimal aufeinander abgestimmt sind. Der Messbecher ist glattwandig und in Zylinderform ausgebildet. Der Umriss des Rührpaddels st rechteckig und stimmt mit dem Längsquerschnitt des glattwandigen Rührbechers im wesentlichen überein. In den Längsseitenwänden des Rührpaddels sind nach außen hin offene Ausnehmungen vorgesehen, die die gerührte Probe durchlassen. Auch in der Innenfläche des Rührpaddels sind Ausnehmungen vorgesehen.

Der derart einfach aufgebaute Probenbecher wird vorzugsweise durch Bajonettverschluss im Gerät gehalten und kann aus beschichtetem Stahlblech oder auch aus Kunststoff bestehen.

Auch das Rührpaddel zeichnet sich durch seinen einfachen kostengünstigen Aufbau aus.

Als Antrieb wird ein bürstenloser und elektronisch drehzahlstabilisierter Motor vorgeschlagen, während im Stand der Technik Wechselstrommotoren mit Kohlebürsten verwendet wurden, bei denen die Drehzahl manuell über Potentiometer eingestellt werden muss und somit keine kontrollierte Regulierung der Drehzahl durch Ist/Sollabgleich während der Messung möglich ist.

Die Messung kann ohne die oben beschriebenen vorbereitenden Handlungen sofort beginnen, so bald sich das Rührpaddel in Bewegung setzt.

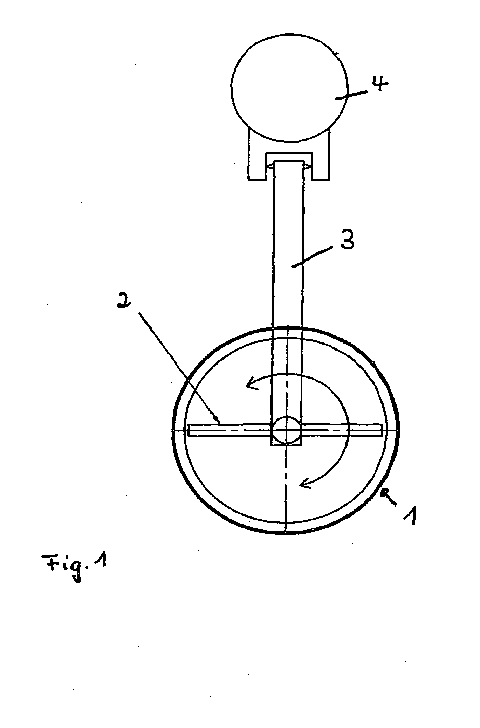

In der Figur 1 ist der schematische Aufbau der Vorrichtung zur Durchführung des erfindungsgemäßen Verfahrens dargestellt.

Mit dem Bezugszeichen 1 ist der glattwandige zylinderförmige Probenbecher bezeichnet, in dem ein Rührpaddel 2 rotiert. Das Rührpaddel 2 wird von einem hier nicht dargestellten Elektromotor angetrieben, der sich oberhalb des Probenbechers 1 befindet. Dieser Elektromotor ist drehbar gelagert und über einen Hebelarm 3 mit einer mit Dehnungsmessstreifen bestückten Messeinrichtung 4 verbunden. Die von den Dehnungsmessstreifen gemessene Kraft ist die Torsionskraft, die aus den dem Rührpaddel von der Probe entgegengebrachten Widerstand erzeugt wird. Die Software berechnet aus dieser auf die Dehnungsmessstreifen ausgeübten Kraft und der definierten Länge des Hebels 3 das entsprechende Drehmoment und zeigt dieses im Diagramm in Abhängigkeit von der Laufzeit an.

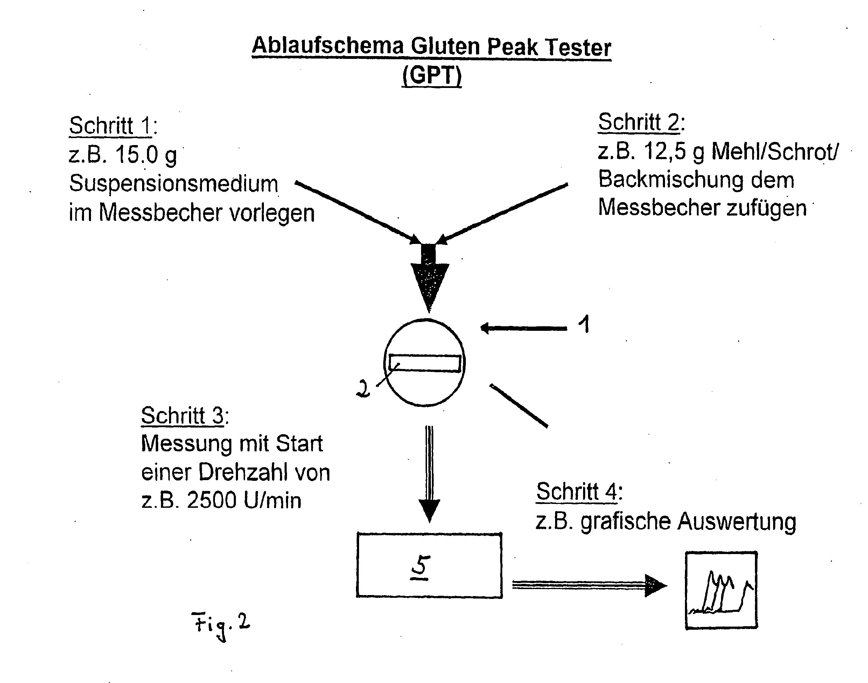

Die Figur 2 zeigt das Ablaufschema des erfindungsgemäßen Verfahrens.

Im ersten Schritt wird in den Probenbecher 1 das Suspensionsmedium (wässrige Lösung mit Salzen, Basen, Säuren) eingegeben. Die Menge beträgt beispielsweise 15 g. Im zweiten Schritt wird das zu testende Mehl, der Schrot, die Backmischung in den Probenbecher 1 gegeben. Die Menge beträgt beispielsweise 12,5 g. Im dritten Schritt beginnt die Messung mit dem Start des Rührpaddels 2 bei einer Drehzahl von beispielsweise 2500 U/min. Es wird nun die Kraft gemessen, daraus das entsprechende Drehmoment errechnet und im vierten Schritt graphisch ausgewertet. Dies geschieht über eine auf einem Computer 5 laufende Software.

## Patentansprüche

1. Verfahren zum Schnelltest der Qualität von Getreiden, Schroten und Mehlen durch Messung der Glutenaggregation, wobei die zu testende Probe in wässriger Lösung/Suspension mit Mineralsalzen und/oder Säuren und/oder Laugen in definierter Menge in einem Probenbecher (1) mittels eines elektrisch angetriebenen Rührpaddels (2) verrührt und der während des Rührvorganges dem Rührpaddel (2) von der Probe entgegen gesetzte Widerstand gemessen wird,
**dadurch gekennzeichnet,**
**dass** über Dehnungsmessstreifen die auf den drehbar gelagerten Elektromotor des Antriebs einwirkenden Torsionskräfte vom Beginn des Rührvorgangs an gemessen werden und die Messung bei Erreichen des Drehmoment-Maximums beendet wird, wobei der Messvorgang durch eine Software überwacht, gesteuert und ausgewertet wird.

2. Verfahren nach Anspruch 1,
**dadurch gekennzeichnet,**
**dass** die Drehzahl des Elektromotors durch die Software vorgegeben und durch ständigen Vergleich der Soll- und Istwerte automatisch korrigiert wird.

3. Verfahren nach Anspruch 2,
**dadurch gekennzeichnet,**
**dass** die Drehzahl < 6000 U/min ist.

4. Verfahren nach einem der Ansprüche 1 bis 2,
**dadurch gekennzeichnet,**
**dass** das Verhältnis von zu testendem Material und Probenlösung zwischen 1:1,2 und 1:1,25 liegt.

5. Verfahren nach einem der Ansprüche 1 bis 4,
**dadurch gekennzeichnet,**
**dass** das Suspensionsmedium eine Leitfähigkeit > 5µS und einen pH-Wert < 2 bis 13 aufweist.

6. Vorrichtung zur Durchführung des Verfahrens nach einem der Ansprüche 1 bis 5, mit einem Probenbecher (1) und einem durch einen Elektromotor angetriebenen Rührpaddel (2) , wobei der Elektromotor und das Rührpaddel (2) oberhalb des Probenbechers (1) angeordnet und das Rührpaddel (2) in den Probenbecher einfahrbar ist, und die von dem Rührpaddel (2) auf den drehbar gelagerten Elektromotor ausgeübte Kraft über einen Hebelarm (3) auf eine mit Dehnungsmessstreifen arbeitende Messeinrichtung (4) übertragen wird
**dadurch gekennzeichnet,**
**dass** eine Sofware zur Überwachnung, Steuerung und Auswertung des Mess- vorgangs verwendet wird, die so eingerichtet ist, dass aus der bekannten Hebellänge und der gemessenen Kraft das Drehmoment errechnet wird, und dass der Längsquerschnitt des glattwandigen zylindrischen Probenbechers (1) mit dem rechteckigen Umriss des Rührpaddels (2) im wesentlichen übereinstimmt, wobei in den Längsseitenwänden des Rührpaddels nach außen hin offene Ausnehmungen und auch in der Innenfläche des Rührpaddels die Probe durchlassende Ausnehmungen vorgesehen sind.

7. Vorrichtung nach Anspruch 6,
**dadurch gekennzeichnet,**
**dass** der Probenbecher (1) eine Vorrichtung zur Temperaturbestimmung aufweist.

## Claims

1. A method for the high-speed testing of the quality of grains, whole grains and flours by measurement of gluten aggregation, wherein the sample to be tested is mixed in an aqueous solution/suspension with mineral salts and/or acids and/or lyes in a defined quantity in a sample beaker (1) by means of an electrically driven horizontal agitator (2) and wherein the resistance exerted during the agitation process against the horizontal agitator (2) by the sample is measured,
**characterised in that**
the torsional forces acting via strain gauges on the rotatably mounted electric motor of the drive are measured from the start of the agitation process and **in that** measurement is terminated when the maximum torque is reached, wherein the measuring process is monitored, controlled and evaluated by software.

2. The method according to Claim 1,
**characterised in that**
the speed of the electric motor is predetermined by the software and is corrected automatically by constant comparison of the theoretical and actual values.

3. The method according to Claim 2,
**characterised in that**
the speed is < 6000 rpm.

4. The method according to any one of Claims 1 to 2,
**characterised in that**
the ratio of the material to be tested and the sample solution is between 1:1.2 and 1:1.25.

5. The method according to any one of Claims 1 to 4,
**characterised in that**
the suspension medium has a conductivity > 5µS and a pH value < 2 to 13.

6. A device for carrying out the method according to Claims 1 to 5, with a sample beaker (1) and a horizontal agitator (2) driven by an electric motor, wherein the electric motor and the horizontal agitator (2) are arranged above the sample beaker (1) and the horizontal agitator (2) can be inserted in the sample beaker, and wherein the force exerted by the horizontal agitator (2) on the rotatably mounted electric motor is transferred via a lever arm (3) to a measuring device (4) operating with strain gauges,
**characterised in that**
software is used for monitoring, controlling and evaluating the measuring process, which software is designed so that the torque is calculated from the known lever length and measured force, and **in that** the longitudinal cross-section of the smooth-walled cylindrical sample beaker (1) roughly corresponds to the rectangular contour of the horizontal agitator (2), wherein recesses open towards the outside and also recesses allowing the sample to pass into the inner surface of the horizontal agitator are provided in the longitudinal side walls of the horizontal agitator.

7. A device according to Claim 6,
**characterised in that**
the sample beaker (1) has a device for temperature determination.

## Revendications

1. Procédé pour tester rapidement la qualité de céréales, de gruaux et de farines en mesurant l'agrégation du gluten, comportant les étapes consistant à agiter l'échantillon à tester dans une solution/suspension aqueuse avec des sels minéraux et/ou des acides et/ou des bases en une quantité définie dans un bécher d'échantillon (1), au moyen d'une pale d'agitation entraînée électriquement, et mesurer la résistance que l'échantillon oppose à l'encontre de la pale d'agitation (2) pendant le processus d'agitation,
**caractérisé en ce que** les forces de torsion qui s'exercent sur le moteur d'entraînement électrique monté de manière rotative sont mesurées au moyen de bandes de mesure d'allongement, dès le début du processus d'agitation, et la mesure s'achève lorsque le couple maximal est atteint, dans lequel le processus de mesure est surveillé, commandé et évalué au moyen d'un logiciel.

2. Procédé selon la revendication 1, **caractérisé en ce que** la vitesse de rotation du moteur électrique est prédéfinie par le logiciel et est automatiquement corrigée en comparant les valeurs de consigne et les valeurs réelles de manière continue.

3. Procédé selon la revendication 2, **caractérisé en ce que** la vitesse de rotation est < à 6 000 t/min.

4. Procédé selon l'une quelconque des revendications 1 à 2, **caractérisé en ce que** le rapport de la matière à tester et la solution d'échantillon est compris entre 1:1,2 et 1:1,25.

5. Procédé selon l'une quelconque des revendications 1 à 4, **caractérisé en ce que** le milieu de suspension a une conductivité > 5 µS et un pH < 2 à 13.

6. Dispositif pour mettre en oeuvre le procédé selon l'une quelconque des revendications 1 à 5, comportant un bécher d'échantillon (1) et une pale d'agitation (2) entraînée par un moteur électrique, dans lequel le moteur électrique et la pale d'agitation (2) sont agencés au-dessus du bécher d'échantillon (1) et la pale d'agitation (2) peut être introduite dans le bécher d'échantillon, et la force que la pale d'agitation (2) exerce sur le moteur électrique monté de manière rotative est transmise par un bras de levier (3) à un dispositif de mesure fonctionnant avec des bandes de mesure d'allongement,
**caractérisé en ce qu'**un logiciel est utilisé pour surveiller, commander et évaluer le processus de mesure, lequel logiciel est configuré de telle sorte que le couple est calculé à partir de la longueur de levier connue et de la force mesurée, et **en ce que** la section transversale longitudinale du bécher d'échantillon cylindrique à paroi plate (1) correspond au contour sensiblement rectangulaire de la pale d'agitation (2), des évidements ouverts vers l'extérieur étant prévus dans les parois latérales longitudinales de la pale d'agitation et des évidements laissant passer l'échantillon étant également prévus dans la surface intérieure de la pale d'agitation.

7. Dispositif selon la revendication 6, **caractérisé en ce que** le bécher d'échantillon (1) comporte un dispositif pour déterminer la température.
